# EUROPÄISCHE PATENTANMELDUNG

(11) **EP 1 179 322 A2**
(43) Veröffentlichungstag der Anmeldung: **13.02.2002**
(21) Anmeldenummer: 01250286.0
(22) Anmeldetag: 06.08.2001
(51) Int. Cl.: A61F 2/06

(54) **Verfahren und Vorrichtung zum Crimpen eines Stents**

(30) Priorität: 09.08.2000 DE 10039617; 19.09.2000 DE 10046528
(71) Anmelder: BIOTRONIK Mess- und Therapiegeräte GmbH & Co Ingenieurbüro Berlin, 12359 Berlin (DE)
(72) Erfinder: Schaldach, Max, Verstorben (DE); Behrend, Detlef, 18119 Rostock (DE); Schmitz, Klaus-Peter, 18055 Rostock (DE); Lootz, Daniel, 18119 Warnemünde (DE); Müller, Heinz, 91054 Erlangen (DE); Esperschidt, Dietmar, 90768 Fürth (DE)
(74) Vertreter: Eisenführ, Speiser & Partner

(57) **Zusammenfassung**

Die Erfindung betrifft ein Verfahren und eine Vorrichtung zum Crimpen eines Stents auf einen Ballon eines Ballonkatheters, wobei der Stent auf dem Ballon derart angeordnet wird, daß die Außenoberfläche des Ballons und die Innenoberfläche des Stent miteinander in Kontakt stehen, um eine Kombination aus Ballon und Stent zu bilden, wobei die Kombination zusammengepreßt wird, um den Stent auf den Ballon zu crimpen. Die Erfindung zeichnet sich dadurch aus, daß die Kombination entlang ihrer Längsachse unterschiedlich stark zusammengepreßt wird.

## Beschreibung

Die Erfindung betrifft ein Verfahren und eine Vorrichtung zum Crimpen eines Stents auf einen Ballon eines Ballonkatheters, wobei der Stent auf dem Ballon derart angeordnet wird, daß die Außenoberfläche des Ballons und die Innenoberfläche des Stent miteinander in Kontakt stehen, um eine Kombination aus Ballon und Stent zu bilden, wobei die Kombination zusammengepreßt wird, um den Stent auf den Ballon zu crimpen.

Derartige Verfahren und Vorrichtungen sind aus dem Stand der Technik bekannt. Sie dienen zum Erzeugen einer Kombination aus dem Ballon eines Ballonkatheters und eines auf dem Ballon gecrimpten Stents. Auf diese Weise wird es möglich, den Stent mit verringertem Außendurchmesser an seiner gewünschten Position auf dem Ballon mit Hilfe des Katheters in den Bereich der zu dilatierenden Stenose zu bringen und gleichzeitig den Stent auf dem Ballon fixiert zu halten.

Bei den aus dem Stand der Technik bekannten Vorrichtungen und Verfahren wird der Stent zum Crimpen auf dem Ballon durch radial nach innen wirkende Kräfte solange komprimiert, bis er fest auf dem Ballon aufsitzt, jedoch immer noch durch Aufblasen des Ballons entfaltet werden kann, um in die Gefäßinnenwand einer Stenose eingepaßt werden zu können, um diese zu stützen. Ein derartiges Verfahren und eine derartige Vorrichtung sind beispielsweise aus der US 5,836,952 bekannt.

Nachteilig bei den aus dem Stand der Technik bekannten Vorrichtungen und Verfahren ist es, daß die Kanten der Stents herausstehen bzw. herausgebogen werden können, wenn der Stent beim Einführen der Kombination durch die Körpergefäße hin zu der Stenose teilweise durch enge Gefäßkrümmungen geführt werden muß. Man nennt dieses Herausstehen, welches insbesondere bei segmentierten Stents auftritt, den sogenannten Fishscaling-Effekt. Dieser Effekt führt bei seinem Auftreten zu Schädigungen der Innenwände der entsprechende Gefäße oder auch zum Verhaken (mit der Gefäßwand) und eventuellem Verschieben oder Abrutschen des Stents auf dem Katheterballon, was wiederum zu Ablagerungen oder Rupturen der Gefäße führen kann.

Aufgabe der vorliegenden Erfindung ist es daher, eine Kombination aus Stent und Ballon bzw. ein Verfahren und eine Vorrichtung zum Crimpen eines Stents auf einem Ballon zur Verfügung zu stellen, welche die vorgenannten Nachteile vermeiden und den Stent sicher an seinen Implantationsort bringen.

Diese Aufgabe wird erfindungsgemäß durch ein Verfahren gemäß Anspruch 1, eine Crimpvorrichtung gemäß Anspruch 5, einen Stent gemäß Anspruch 9 und Kombinationen gemäß den Ansprüchen 8 und 10 gelöst.

Die Vorteile der vorliegenden Erfindung liegen insbesondere darin, daß der Fishscaling-Effekt dadurch vermindert oder vermieden werden kann, daß die beiden axialen Enden der Stents nach innen gebogen werden. Dadurch liegen insbesondere die Kanten von in axialer Richtung des Stents hintereinander angeordneten Segmenten des Stents auch bei dem Einführen eines die Kombination tragenden Katheters durch enge Gefäßwindungen am Ballon an. Die Gefahr der Verletzung der Gefäßinnenwand wird somit ebenso wie die zum Einführen eines derartigen Ballonkatheters bzw. einer derartigen Kombination erforderlichen Schubkraft vermindert. Darüber hinaus ist es weiterhin vorteilhaft, daß aufgrund der Erfindung die Haftung zwischen dem Stent und dem Ballon vergrößert wird, so daß ein versehentliches Herabgleiten des Stents vom Ballon beim Einführen der Kombination in das Körpergefäß vermieden wird.

Vorteilhafte Ausführungsformen des erfindungsgemäßen Verfahrens bzw. der erfindungsgemäßen Vorrichtung verwenden Walzen bzw. Platten, die einen alternierend zu- und abnehmenden Abstand zueinander aufweisen, um eine zwischen den Walzen bzw. Platten eingeklemmte und zusammengepreßte Kombination aus Stent und Ballon entlang deren Längsachse unterschiedlich stark zu crimpen.

Besonders vorteilhaft läßt sich die Erfindung realisieren, indem die Walzen oder Platten mit einer geriffelten Oberfläche versehen werden. Wird nun eine Kombination aus Stent und Ballon zwischen derartige Walzen hindurchgezwängt, so wird der Stent von den Wellenkämmen der Riffelung stärker auf den Ballon gecrimpt als von den Wellentälern der Riffelung. Gleiches gilt für ein Zusammenpressen mit Hilfe zweier parallel angeordneter Platten. Die einander zugewandten Oberflächen der Platten sind dabei ebenfalls in regelmäßiger Abfolge wellenförmig geriffelt. Zum Crimpen der Stents werden die beiden Platten gleichmäßig gegeneinander verschoben gleichzeitig aufeinander zu bewegt, wodurch die Kombination aus Stent und Ballon zusammengepreßt und somit der Stent auf den Ballon gecrimpt wird.

Alternativ können auch spezielle Profilwalzen in 2-, 3- oder Mehrpunktwalzvorrichtungen, gerade wie auch schräg, zur Erzeugung der Crimpung verwendet werden. Weiter alternativ lassen sich für die Erfindung Spannzangen mit speziell geformten Innenfutter oder auch entsprechend dem gewünschten Crimpprofil geformte Stempel verwenden. Schließlich läßt sich das erfinderische unregelmäßige Crimpen auch mit dem lokalen Umschließen des Stent mit aperturähnlichen Blenden erzielen.

Eine weitere vorteilhafte Ausführungsform der erfindungsgemäßen Crimpvorrichtung zeichnet sich dadurch aus, dass die Stent-Katheter-Kombination zum Crimpen in einem Zwickel zwischen einer ersten Walze und einer zweiten, benachbart und parallel zu der ersten Walze angeordneten Walze positioniert wird, und dort mit Hilfe einer dritten, im wesentlich parallel zu den beiden anderen Walzen angeordneten Walze in dem Zwickel zusammengepreßt wird, wobei mindestens eine der Walzen mit einer geriffelten Oberfläche versehen ist. Zu diesem Zweck wird bevorzugt die dritte Walze um ihre Längsachse rotierend angetrieben. In bevorzugter Ausführungsform sind die erste und die zweite Walze starr gelagert, während die dritte Walze um die erste Walze mit Hilfe eines die erste und die dritte Walze verbindenen Hebels schwenkbar gelagert ist, um so die in dem Zwickel zwischen erster und zweiter Walze positionierte Stent-Katheter-Kombination durch Verschwenken des Hebels und der dritten Walze in dem Zwickel zusammen zu drücken und somit zu crimpen.

Weitere vorteilhafte Ausführungsformen der Erfindung sind in den Unteransprüchen angegeben.

Ausführungsbeispiele der Erfindung werden im folgenden anhand der Zeichnung beschrieben.

### Die Figuren der Zeichnung zeigen:

- Fig. 1: eine erfindungsgemäße Crimpvorrichtung in einer ersten Ausführungsform;
- Fig. 1a: eine Vergrößerung eines Teils der Fig. 1;
- Fig. 2: eine erfindungsgemäße Crimpvorrichtung in einer zweiten Ausführungsform;
- Fig. 3: eine erfindungsgemäße Kombination aus Stent und Ballon;
- Fig. 4: die Kombination aus Fig. 3 bei Krümmung derselben;
- Fig. 5: eine weitere Ausführungsform einer Crimpvorrichtung;
- Fig. 6: eine weitere Ausführungsform einer Crimpvorrichtung; und
- Fig. 7: die Ausführungsform der Fig. 6 im Querschnitt.

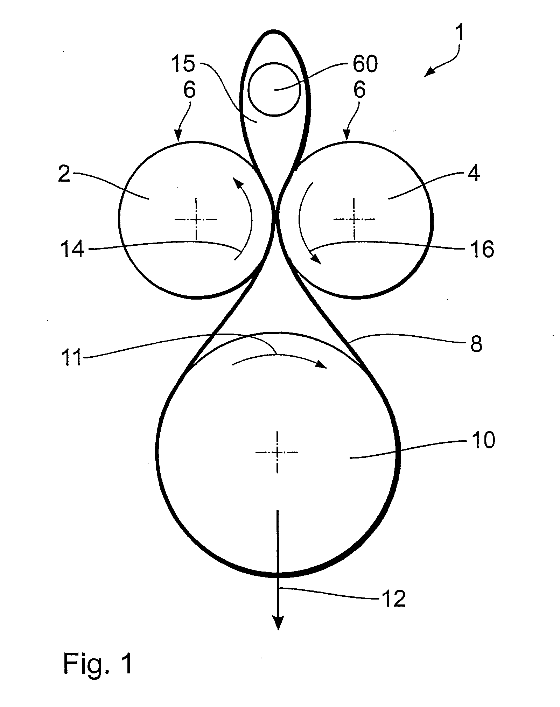

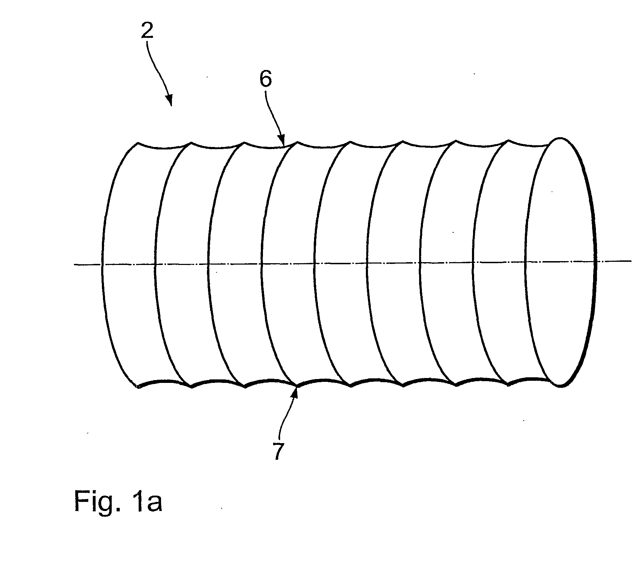

Gemäß Fig. 1 wird bei einer ersten Ausführungsform 1 einer Crimpvorrichtung eine Stent-Katheterkombination 60 in eine Schlaufe eines umlaufenden Endlosbandes 8 gelegt. Dieses Band 8 läuft zwischen zwei Walzen 2 und 4, die der in Figur 1a dargestellten Rolle 2 entsprechen. Die Walzen weisen jeweils umlaufende Rippen 7 auf ihren Oberflächen 6 auf. Sie sind auf starren Achsen derart gelagert, daß nur ein geringer Spalt zwischen den Walzen existiert, der verhindert, daß die Stent-Katheterkombination 60 zwischen die Walzen 2, 4 gerät. Die Antriebswalze 10 bringt das Endlosband 8 in Rotation. Durch diese Rotation 11 werden die Walzen 2 und 4 im Sinne der Pfeile 14 und 16 in Drehung versetzt. Gleichzeitig wird die Antriebswalze 10 mit einer Kraft 12 beaufschlagt, die das Band 8 spannt und so die Stent-Katheterkombination 60 in den Zwischenraum 15 zwischen den Walzen 2 und 4 drückt. So wird das Rillenprofil 6 der Walzen 2 und 4 auf die Stent-Katheterkombination 60 übertragen.

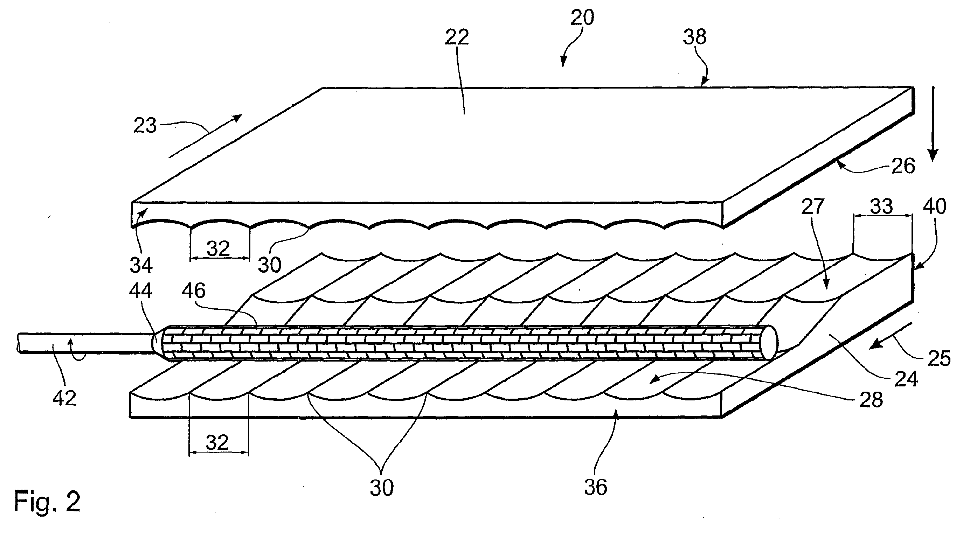

Fig. 2 zeigt eine Crimpvorrichtung in einer zweiten Ausführungsform 20. Als Preßmittel weist die Crimpvorrichtung 20 eine obere Platte 22 und eine im wesentlichen parallel zu der oberen Platte 22 angeordnete untere Platte 24 auf. Die einander zugewandten Oberflächen 26 bzw. 28 der Platten 22 und 24 weisen die in der Fig. 2 dargestellte Riffelung auf, bei der die Oberfläche 26 bzw. 28 im wesentlichen wellenförmig ausgebildet ist, jedoch die Wellenkämme 30 spitz zulaufend ausgebildet sind. Der Abstand 32 der Wellenkämme 30 an den Seiten 34 bzw. 36 ist gleich dem Abstand 33 der Wellenkämme 30 an den Rückseiten 38 bzw. 40 der Platten 22 bzw. 24. Das Crimpen geschieht, indem die geriffelten Platten 22 und 24 gemäß den Pfeilen 23 und 25 gegeneinander verschoben. Durch den in der unteren Platte 24 vorgesehenen Absatz 27 verringert sich der Abstand der Platten 22 und 24 während ihrer gegenläufigen Bewegung, so daß es nicht notwendig ist, daß die Platten 22, 24 während der Bewegung auch aufeinander zu bewegt werden.

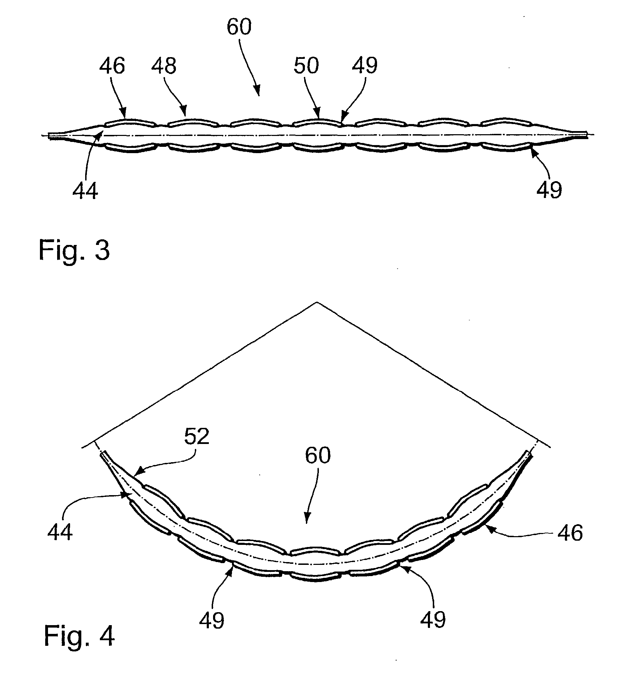

Alternativ kann der Abstand 32 der Wellenkämme 30 von den Vorderseiten 34 bzw. 36 der Platten 22 bzw. 24 zu dem Abstand 33 an den Rückseiten 38 bzw. 40 der Platten 22 bzw. 24 zunehmen, da bei der Kompression des zwischen den Platten 22 und 24 einzuklemmenden Ballonkatheters 42 mit dem auf einen Ballon 44 angeordneten Stent 46 der Stent 46 verlängert wird. Auf diese Weise wird bei dieser alternativen Ausführungsform sichergestellt, daß die Wellenkämme 30, d.h. die Stellen verstärkten Crimpens, an gleichbleibender Stelle auf dem Stent 46 auf den Stent 46 einwirken. Bei dem Stent 46 handelt es sich bevorzugt um einen Segmente aufweisenden Stent 46 (siehe auch Fig. 4).

Fig. 3 zeigt einen mit Hilfe der Crimpvorrichtung 20 gemäß Fig. 2 hergestellte Kombination 60 aus einem Stent 46 und einem Ballon 44, wobei der Stent Segmente 48 aufweist. Die Fig. 3 verdeutlicht, daß die Ränder 49 stärker auf den Ballon 44 gecrimpt sind als der zentrale Bereich 50 der Segmente 48, so daß ein wellenförmiges Profil des Stent 46 entsteht. Handelt es sich bei dem Stent 46 alternativ um einen nicht-segmentierten Stent, so kann durch entsprechende Ausbildung der Oberflächen der Walzen 2, 4 (Fig. 1) oder Platten 22, 24 (Fig. 2) auf deren Mantelfläche ein schuppenförmiges Profil erzeugt werden, so daß das Gleiten eines derartigen Stent im Gefäß verbessert wird.

Fig. 4 verdeutlicht die Wirkung der vorliegenden Erfindung. Bei Krümmung der in Fig. 3 dargestellten Kombination 60 aus Ballon 44 und Stent 46 stehen die Ränder 49 der Segmente 48 nicht - wie im Stand der Technik - von der Oberfläche 52 des Ballons 44 ab; vielmehr verbleiben die Ränder 49 der Segmente 48 an der Oberfläche 52 des Ballons 44, so daß Verletzungen der Gefäße beim Einführen einer derartigen Kombination 60 vermieden werden.

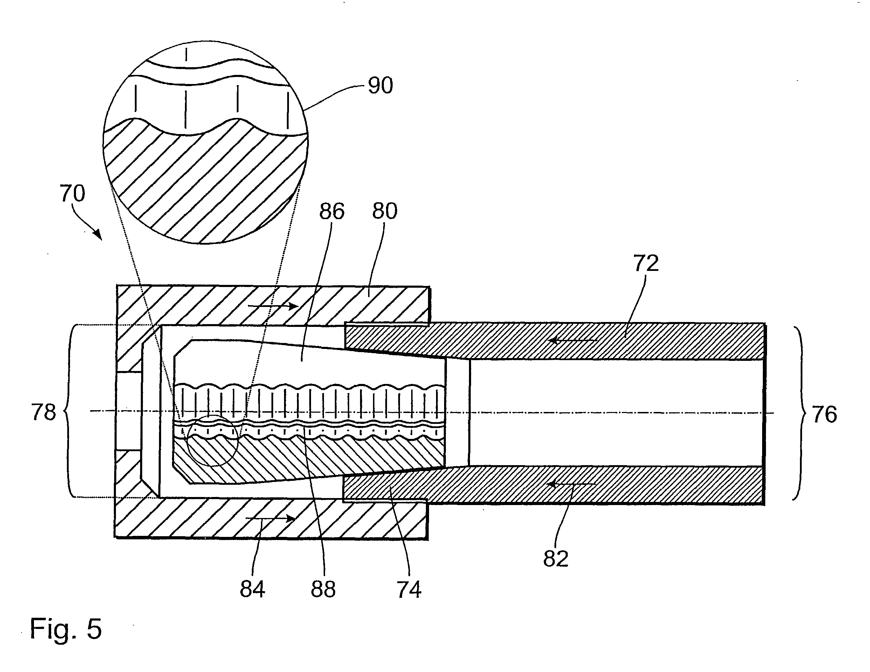

Fig. 5 zeigt eine weitere Ausführungsform 70 einer erfindungsgemäßen Crimpvorrichtung. Die Crimpvorrichtung 70 wird im wesentlichen durch eine Spannzange gebildet. Die Spannzange besteht aus einem Hohlzylinder 72, der an seinem in der Fig. 5 links dargestellten Ende 74 eine sich stetig verjüngende Wandstärke aufweist. Der Außendurchmesser 76 des Hohlzylinders 72 ist dem Innendurchmesser 78 eines zweiten Hohlzylinders 80 angepaßt, so daß der Hohlzylinder 72 in den zweiten Hohlzylinder 80 gemäß den Pfeilen 82 eingeführt werden kann. Ebenso kann der zweite Hohlzylinder 80 gemäß den Pfeilen 84 auf den ersten Hohlzylinder 72 aufgeschoben werden.

Die angeschrägte Innenwandung am Ende 74 des Hohlzylinders 72 ist dem Profil von Backen 86 angepaßt, die sich in dem Hohlzylinder 80 befinden. Die Backen 86 sind in der Art eines 3-, 4- oder Mehrbackenfutters ausgebildet, wie sie aus Bohr- oder Drehmaschinen bekannt sind. Durch die Bewegung des Hohlzylinders 72 in den Hohlzylinder 80 werden die Backen 86 zusammengedrückt und ein sich innerhalb des Hohlraumes 88 zwischen den Backen befindende Stentkatheterkombination wird auf diese Weise mit Hilfe der Backen 86 gecrimpt.

Der Ausschnitt 90 zeigt vergrößert die wellige Innenoberfläche der Backen 86, so daß der Stent mit entlang seiner Längsachse alternierender Stärke gecrimpt wird.

Besonders vorteilhaft ist es, wenn der Stent in zwei Stufen gecrimpt wird, d.h. zunächst zur Erzeugung einer großen Durchmesserveränderung mit einer Crimpvorrichtung 1 gemäß Fig. 1 und dann zur Erzeugung einer feinen Durchmesseränderung mit einer Spannzange 70 gemäß Fig. 5.

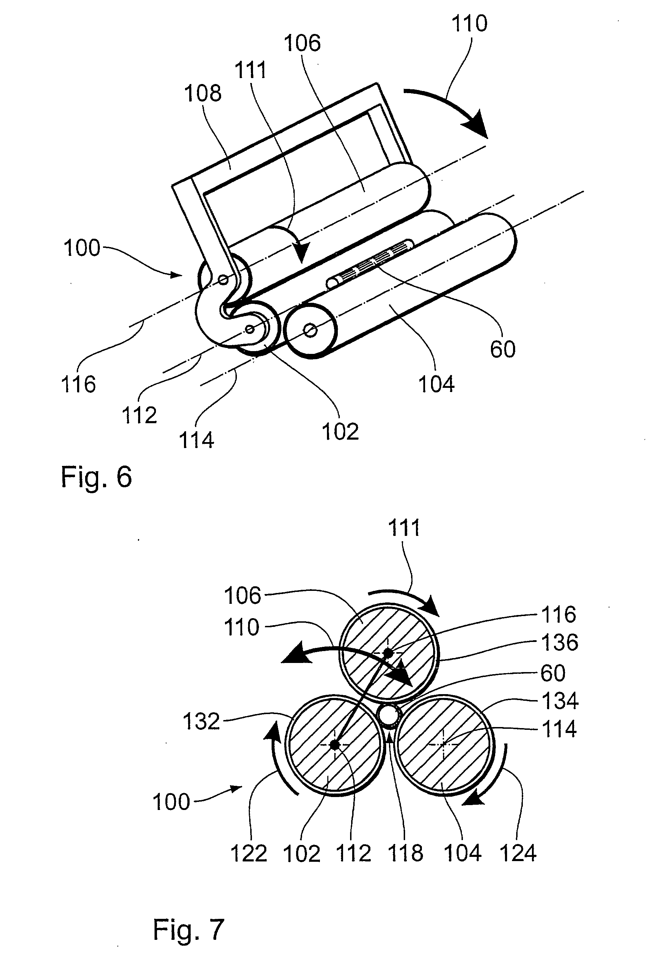

Fig. 6 zeigt eine Crimpvorrichtung in einer weiteren Ausführungsform 100. Die Crimpvorrichtung 100 ist in der Fig. 6 in einer perspektivischen Ansicht dargestellt. Die Crimpvorrichtung 100 weist drei Walzen 102, 104 und 106 auf. Die Walzen 102, 104, 106 sind mit ihren Längsachsen 112, 114 bzw. 116 parallel zueinander angeordnet. Dabei sind die Walzen 102 und 104 starr gelagert. Die Walze 106 ist über einen Schwenkhebel 108 mit der Walze 102 verbunden und mit Hilfe des Schwenkhebels 108 um die Achse 112 der Walze 102 in der durch den Pfeil 110 angedeuteten Richtung schwenkbar. Der Abstand zwischen den Walzen 102 und 104 ist so gewählt, dass die Stent-Katheter-Kombination 60 auch im gecrimpten Zustand nicht durch den Zwischenraum zwischen den Walzen 102, 104 fallen kann. Weiterhin ist die Walze 106 mit einem nicht dargestellten Antrieb verbunden, um sie in Richtung des Pfeils 111 in Rotation um ihre Längsachse 116 zu versetzen.

Die Arbeitsweise der Crimpvorrichtung 100 sei anhand der Fig. 7 beschrieben. Die Fig. 7 zeigt die Crimpvorrichtung 100 der Fig. 6 im Querschnitt. Teile, die denen der Fig. 6 entsprechen, sind in der Fig. 7 mit den gleichen Bezugszeichen bezeichnet. Um die Stent-Katheter-Kombination 60 zu crimpen, wird diese in den Zwickel 118 zwischen den Walzen 102 und 104 gelegt. Anschließend wird die Walze 106 mit Hilfe des in der Fig. 6 dargestellten Hebels 108 um die Längsachse 112 der Walze 102 gemäß dem Pfeil 110 verschwenkt, um so die Stent-Katheter-Kombination 60 in den Zwickel 118 zwischen den Walzen 102 und 104 zu drücken. Gleichzeitig wird die Walze 106 mit Hilfe eines nicht dargestellten Antriebes gemäß Pfeil 111 in Rotation um ihre Längsachse 116 versetzt. Auf diese Weise wird die Stent-Katheter-Kombination 60 in Rotation versetzt, wodurch wiederum die Walzen 102 und 104 um ihre Längsachsen 112 bzw. 114 ebenfalls gemäß der Pfeile 122 bzw. 124 in Rotation versetzt werden. Durch die durch die Walze 106 ausgeübte Kraft wird die Stent-Katheter-Kombination 60 in dem Zwickel 118 zwischen den Oberflächen 132, 134 und 136 der Walzen 102, 104 bzw. 106 zusammengepreßt. Um die Stent-Katheter-Kombination 60 entlang ihrer Längsachse unterschiedlich stark zu crimpen, sind die Oberflächen 132, 134 bzw. 136 der Walzen 102, 104 und 106 gemäß der in der Fig. 1a dargestellten Art geriffelt ausgebildet.

Die in den Figuren 6 und 7 dargestellte Ausführungsform 100 der erfindungsgemäßen Crimpvorrichtung hat den Vorteil, dass mit ihrer Hilfe insbesondere Koronarstents, die gegenüber peripheren Stents ein filigraneres Design aufweisen, problemlos gecrimpt werden können, ohne daß zu große Kräfte auf den Koronarstent ausgeübt werden.

## Patentansprüche

1. Verfahren zum Crimpen eines Stents (46) auf einen Ballon (44) eines Ballonkatheters (42), mit den Schritten:
- der Stent (46) wird auf dem Ballon (44) derart angeordnet, daß die Außenoberfläche (52) des Ballons (44) und die Innenoberfläche des Stents (46) miteinander in Kontakt stehen, um eine Kombination (10, 10', 60) aus Ballon (44) und Stent (46 zu bilden,
- die Kombination (10, 10', 60) wird zusammengepreßt, um den Stent (46) auf den Ballon (44) zu crimpen,
**dadurch gekennzeichnet, daß** die Kombination (10, 10', 60) entlang ihrer Längsachse unterschiedlich stark zusammengepreßt wird.

2. Verfahren nach Anspruch 1, bei dem die Kombination (10, 10', 60) zum Crimpen in einem zwischen zwei Walzen (2, 4) eingeklemmten Band (8) geführt und mit dem Band (8) in den Zwickel (15) der Walzen (2,4) gezogen und dort zusammengepreßt wird, wobei die Walzen (2, 4) entlang ihrer Rotationsachsen einen alternierend zu- und abnehmenden Abstand zueinander aufweisen.

3. Verfahren nach Anspruch 1, bei dem die Kombination (10, 10', 60) zwischen zwei im wesentlichen parallel zueinander angeordneten Platten (22, 24) geführt und zwischen diesen zusammengepreßt wird, indem die Platten (22, 24) gegeneinander verschoben werden.

4. Verfahren nach Anspruch 3, wobei die Platten (22, 24) senkrecht zu der Verschieberichtung einen alternierend zu- und abnehmenden Abstand zueinander aufweisen.

5. Verfahren nach Anspruch 1, bei dem die Kombination (60) zum Crimpen in einen Zwickel (118) zwischen einer ersten Walze (102) und einer zweiten, benachbart und parallel zu der ersten Walze (102) angeordneten Walze (104) positioniert wird, und in dem Zwickel (118) mit Hilfe einer dritten, im wesentlichen parallel zu ersten (102) und der zweiten Walze (104) angeorndeten Walze (106) zusammengepreßt wird, wobei mindestens eine Walze (102, 104, 106) mit einer geriffelten Oberfläche (132, 134, 136) versehen ist.

6. Verfahren nach Anspruch 5, wobei eine, bevorzugt die dritte (106) Walze (102, 104, 106) zur Erzeugung einer Rotationsbewegung um ihre Längsachse (112, 114, 116) angetrieben wird.

7. Crimpvorrichtung zum Crimpen eines Stents (46) auf einen Ballon (44) eines Ballonkatheters (42), mit einem zwei einander zugeordnete Teile (2, 4, 22, 24) aufweisenden Preßmittel zum Aufbringen von radial auf eine zwischen den Teilen (2, 4, 22, 24) angeordnete Kombination (10, 10', 60) aus Ballon (44) und Stent (46) wirkenden Kräften, bei welcher Kombination (10, 10', 60) der Stent (46) auf dem Ballon (44) derart angeordnet ist, daß die Außenoberfläche (52) des Ballons (44) und die Innenoberfläche des Stents (46) miteinander in Kontakt stehen, um die Kombination (10, 10', 60) zu bilden, um die Kombination (10, 10', 60) zusammenzupressen und den Stent (46) auf den Ballon (44) zu crimpen, **dadurch gekennzeichnet, daß** die beiden Teile (2, 4, 22, 24) entlang der Längsachse der zwischen ihnen angeordneten Kombination (10, 10', 60) einen alternierend zu- und abnehmenden Abstand aufweisen.

8. Vorrichtung nach Anspruch 7, wobei die beiden Teile (2, 4, 22, 24) entlang ihres Umfanges mit einer geriffelten Oberfläche versehene, mit ihren Rotationsachsen im wesentlichen parallel zueinander angeordnete Walzen (2, 4) sind, so daß die Kombination (10, 10', 60) mittels eines zwischen den Walzen (2, 4) eingeklemmten Bandes (8) geführt in den Zwickel (15) der Walzen (2, 4) hineingezogen und dort zusammengepreßt werden kann.

9. Vorrichtung nach Anspruch 7, wobei die beiden Teile (2, 4, 22, 24) auf ihren einander zugeordneten Seiten mit einer geriffelten Oberfläche (26, 28) versehene, im wesentlichen parallel zueinander angeordnete Platten (22, 24) sind, so daß die Kombination (10, 10', 60) zwischen den Platten (22, 24) geführt und zwischen diesen zusammengepreßt werden kann.

10. Vorrichtung nach Anspruch 7, wobei die beiden Teile Walzen (102, 104, 106) sind, die mit ihren Rotationsachsen (112, 114, 116) im wesentlichen parallel zueinander angeordnet sind, mindestens eine der Walzen (102,104, 106) auf ihrem Umfang mit einer geriffelten Oberfläche (132, 134, 136) versehen ist, und eine dritte Walze (106) vorgesehen ist, mit deren Hilfe die Kombination (60) in einem Zwickel (118) zwischen der ersten Walze (102) und der zweiten Walze (104) preßbar ist.

11. Vorrichtung nach Anspruch 10, wobei die Achsen (112,114,116) während des Crimpens der Kombination (60) die Spitzen eines im wesentlichen gleichschenkligen Dreiecks bilden.

12. Vorrichtung nach einem der Ansprüche 10 oder 11, wobei die erste Walze (104) und die zweite Walze (104) mit einem Abstand zueinander angeordnet sind, und der Abstand kleiner ist als der Durchmesser der Kombination (60) im gecrimpten Zustand.

13. Vorrichtung nach einem der Ansprüche 11 bis 12, wobei mindestens eine der Walzen (102, 104, 106), bevorzugt die dritte Walze (106), mit einem Antrieb zur Erzeugung einer Rotationsbewegung um ihre Längsachse (116) verbunden ist.

14. Vorrichtung nach einem der Ansprüche 11 bis 13, wobei ein Anschlag zur definierten Positionierung der Kombination (60) in der Vorrichtung (100) vorgesehen ist.

15. Kombination aus einem Ballon (44) und einem auf diesen gecrimpten Stent (46), **dadurch gekennzeichnet, daß** der Stent (46) entlang seiner Längsachse unterschiedlich stark auf den Ballon (44) gecrimpt ist.

16. Stent, mit mehreren, in axialer Richtung hintereinander angeordneten, Ränder (49) und einen zentralen Bereich (50) aufweisenden Segmenten (48), wobei die Ränder (49) der Segmente (48) stärker gecrimpt sind als deren zentrale Bereiche (50).

17. Stent, mit einem dünnwandigen Hohlzylinder, dessen Mantelfläche netzförmig ausgebildet ist, wobei die Enden des Hohlzylinders stärker gecrimpt sind als die Mantelfläche.

18. Kombination nach Anspruch 15, mit einem Stent (46) nach einem der Ansprüche 1 6 oder 17.
